# EUROPEAN PATENT SPECIFICATION

(11) **EP 3 426 204 B1**
(45) Date of publication and mention of the grant of the patent: **20.11.2019**
(21) Application number: 17716069.4
(22) Date of filing: 10.03.2017
(51) Int. Cl.: A61F 9/00, A61M 5/34

(54) **FLUID APPLICATOR FOR USE IN OPHTHALMIC APPLICATIONS**
FLUIDAPPLIKATOR ZUR VERWENDUNG IN OPHTHALMISCHEN ANWENDUNGEN
APPLICATEUR DE FLUIDE DESTINÉ À ÊTRE UTILISÉ DANS DES APPLICATIONS OPHTALMIQUES

(30) Priority: 11.03.2016 NL 2016420; 09.05.2016 NL 2016741
(43) Date of publication of application: 16.01.2019
(73) Proprietor: Crea IP B.V., 3237 MG Vierpolders (NL)
(72) Inventor: DAM-HUISMAN, Adriaantje Coliene, 2614 EZ Delft (NL); ECKARDT, Claus Ferdinand, 61350 Bad Homburg (DE); MATTHEIJER, Joost, 3061 CP Rotterdam (NL)
(74) Representative: Nederlandsch Octrooibureau
(86) International application number: PCT/NL2017/050151
(87) International publication number: WO 2017/155405

(56) References cited:
- EP-A1- 2 982 401
- WO-A1-2014/142655
- WO-A2-2004/073873
- DE-A1- 3 328 366
- GB-A- 1 097 307
- US-A1- 2008 306 448
- US-A1- 2012 238 962

## Description

### Field of the invention

The present invention relates to a fluid applicator for use in ophthalmic applications.

### Prior art

International application WO 2015/136035 discloses a syringe for the injection of a surgical auxiliary fluid into an eye. The syringe comprises an injector having a receiving region for receiving a fluid component of the auxiliary fluid, wherein the injector comprises a mixing device for generating the auxiliary fluid. The mixing device comprises a first receiving region with a viscoelastic fluid forming a first fluid component of the auxiliary fluid and further comprises a second receiving region with a dye forming a second fluid component of the auxiliary fluid. The mixing device is configured for mixing the viscoelastic fluid and the dye into the auxiliary fluid. In an embodiment the injector comprise a balloon squeezable by manual actuation for mixing the dye and viscoelastic fluid, and for injecting the mixed fluid.

European patent application EP-A-0 400 216 discloses a single use disposable syringe. In the syringe, a reservoir is formed of two sheets of thermoplastic material having expanded central portions that form a pair of convex domes. Both of the domes are compressible, and a connecting member spaces apart the domes. In response to complete compression of both of the central portions together, the reservoir substantially collapses and each of the domes becomes concave to prevent the reservoir from being reformed and to prevent reuse of the syringe. After partial compression of both of the central portions together and partial collapse of the reservoir, the central portions expand to reform the reservoir and draw fluid into the reservoir, thereby aspirating a hypodermic needle.

American patent publication US2008/306448 discloses a single-handed syringe including a needle, a syringe body and a syringe adapter. The syringe adapter has a reservoir and a plunger moving into and out of the reservoir in a direction perpendicular to a longitudinal direction of the needle.

European patent publication EP-A-2 982 401 discloses a drug injection syringe having a reduced dead volume allowing to inject small amount of a drug without leakage and without an amount of remaining drug.

British patent publication GB-A-1097307 discloses an injection syringe having a needle connected to an external container, which encloses an inner plastic bag. The inner plastic bag may be emptied or filled by pressing, respectively releasing a flexible part of the external container.

German patent publication DE-A-3328366 discloses a syringe with a reservoir having a stiff lower chamber part and a deformable upper chamber part. The deformable upper part allows to empty the reservoir.

American patent publication US2014/0163363 discloses an applicator in the form of a membrane visualization instrument, having a needle 220, a visualization fluid guide 210 in the form of a rigid tube, and a flow control mechanism 100, e.g. a squeezable balloon.

An ophthalmic fluid applicator according to the preamble of claim 1 is known from US-A-2012/0238962.

### Summary of the invention

The present invention seeks to provide an improved fluid applicator, in particular a fluid applicator for use in ophthalmic applications, wherein the fluid applicator allows for steady, precise and accurate manual handling when applying a fluid. For example, the fluid applicator allows precise and accurate manual injection of a fluid into e.g. a vitreous chamber of an eye at any desired angle and/or depth during an ophthalmic procedure. The fluid applicator can also be conveniently refilled using a syringe or any other suitable fluid supply device, e.g. a fluid pouch or the like.

According to the present invention, a fluid applicator is provided comprising an inlet end for connection to a fluid supply device (e.g. a syringe), an outlet end for attachment to a needle element, and a main tubular container body connecting the inlet end and the outlet end; wherein the main tubular container body comprises at least in part a flexible wall portion providing a variable inner volume to the tubular container body.

The flexible wall portion of the tubular container body allows precise manual application of a fluid contained in the variable inner volume during a procedure wherein a syringe needle is attached to the outlet end. In particular, the flexible wall portion of main tubular container body can be operated using e.g. fingertips (e.g. index finger and thumb) for manual compression of the inner volume and provides sensitive tactile feedback from the fluid applicator for sensing ejection intensity. The fluid applicator also provides sensitive tactile feedback for precise and accurate motion control of insertion depth and/or angle of an attached needle during actual use.

The outlet end optionally comprises an outlet one-way valve. The outlet one-way valve is arranged in the outlet end prevents back flow into the fluid applicator and as such prevents back flow and unwanted suction at the needle tip during delicate and precise fluid application.

The fluid applicator of the present invention can therefore be envisaged as a small sized fluid applicator allowing comfortable, precise and accurate manual control of a needle attached thereto, providing excellent tactile feedback comparable to handling an ordinary pencil so that cumbersome and unstable handling of the fluid applicator using a regular syringe configuration is circumvented.

The inlet end comprises an inlet one-way valve preventing back flow into the fluid supply device. This configuration also has the effect that in actual use, fluid can remain in the fluid supply device, as after actuation of the flexible wall portion the resilient action creates an under pressure and sucks in additional fluid possible left in the fluid supply device via the inlet one-way valve.

The main tubular container body comprises a longitudinally arranged rigid wall portion connecting the inlet end and the outlet end, wherein the flexible wall portion is longitudinally arranged between the inlet end and the outlet end.

This allows reliable handling of the fluid applicator, yet still provides for a flexible and compressible longitudinal portion to actuate the fluid applicator.

In yet a further group of embodiments, the fluid applicator further comprises a squeeze actuator, the squeeze actuator comprising a fluid applicator retaining element and an actuator element, This allows a user to more easily exert a force on the flexible wall portion, which allows to easier control the application of fluid, even when this fluid is highly viscous, such as a gel used for subchoroidal buckling.

### Brief description of the figures

The present invention will now be described in more detail by means of a number of exemplary embodiments and with reference to the attached drawings, in which:
Figure 1 shows a three dimensional view of an embodiment of a fluid applicator according to the present invention;
Figure 2 shows a cross sectional view of an embodiment of a main tubular container body as used for the fluid applicator according to the present invention; and
Figure 3 shows a cross sectional view of an embodiment of an inner tubular container body as used for the fluid applicator according to the present invention;
Figure 4 shows a cross sectional view of a further embodiment of the fluid applicator according to the present invention;
Figure 5 shows a perspective view of a further embodiment of the fluid applicator according to the present invention having a squeeze actuator;
Figure 6 shows a cross sectional view of the fluid applicator of Figure 5; and
Figure 7 shows a cross sectional view of an alternative embodiment of the fluid applicator of Figure 5.

### Detailed description of exemplary embodiments.

Fluid applicators in the form of a syringe and needle combination come in various shapes and sizes but most of them do not allow for steady, precise and accurate manual control for ejecting a fluid. For example, most prior art syringe applicators comprise a linearly movable syringe barrel to be operated by means of a thumb for ejecting a fluid from the syringe. However, the way in which such existing syringes are held in one's hand does not promote steady, precise and accurate control of insertion depth and/or insertion angle of the needle. Such syringes are therefore less suitable for delicate procedures often found in ophthalmology for example, wherein the most precise and minute control is required for injecting a fluid, such as ejecting dyes or perfluorocarbons.

In light of the above drawbacks there is a need for a fluid applicator that allows for steady, precise and accurate manual control of a fluid to be ejected through the needle tip. Furthermore, there is a need for a fluid applicator that provides good tactile feedback when used by facilitating manual handling in a natural and familiar way without requiring substantial training of a user.

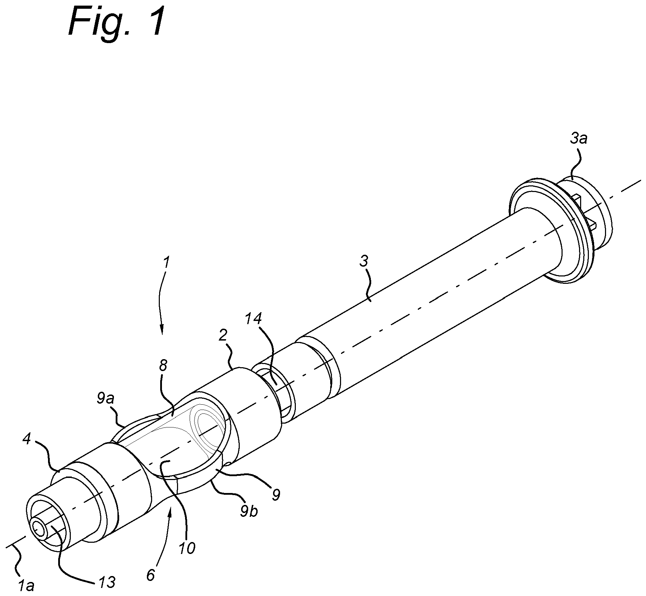

Figure 1 shows a three dimensional view of an embodiment of a fluid applicator according to the present invention. In the embodiment shown, the fluid applicator 1 comprises an inlet end 2 for connection to a fluid supply device 3, an outlet end 4 for attachment to a needle element, and a main tubular container body 6 connecting the inlet end 2 and the outlet end 4. In an advantageous embodiment the main tubular container body is an (elongated) tubular container body 6, providing a pencil-like tactile experience when using the fluid applicator 1.

The fluid supply device 3 can be a standard syringe attached to the inlet end 2, so that by moving a syringe barrel 3a a fluid of choice can be injected into the fluid applicator 1. Alternatively, the fluid supply device 3 is a storage container or a storage bag. In an advantageous embodiment the inlet end 2 and/or the outlet end 4 may each comprises a Luer type connector 13, 14, so that convenient attachment of the needle element and/or the fluid supply device 3 is possible.

The main tubular container body 6 comprises at least in part a flexible, resilient and/or compressible wall portion 8 providing a variable inner volume 10 to the tubular container body 6. Advantageously, the wall portion 8 can be deformed when subjected to manual pressure using fingertips for example. In all embodiments the flexible wall portion 8 is deformable when pressed laterally with respect to a longitudinal axis 1a of the fluid applicator 1. The fluid applicator 1, in particular the outlet end 4 thereof, comprises an optional outlet one-way valve 5 (e.g. a check valve) in this embodiment. It is noted that also an embodiment is envisaged wherein the outlet end 4 is an open connection end.

According to the present invention the fluid applicator 1 is a small sized, e.g. pencil sized, fluid applicator for optimal grip between fingertips, allowing steady, precise and accurate handling for ejecting a fluid from the fluid applicator 1 by pressing the flexible wall portion 8. Furthermore, the fluid applicator 1 provides excellent tactile feedback due to its small, pencil sized shape, offering a natural and familiar gripping experience to a user. The outlet one-way valve 5 in the outlet end 4 of the fluid applicator 1 prevents back flow of fluid into the inner volume 10, so that suction at an end of an attached needle element to the outlet end 4 is prevented and minute control of ejected volumes is provided.

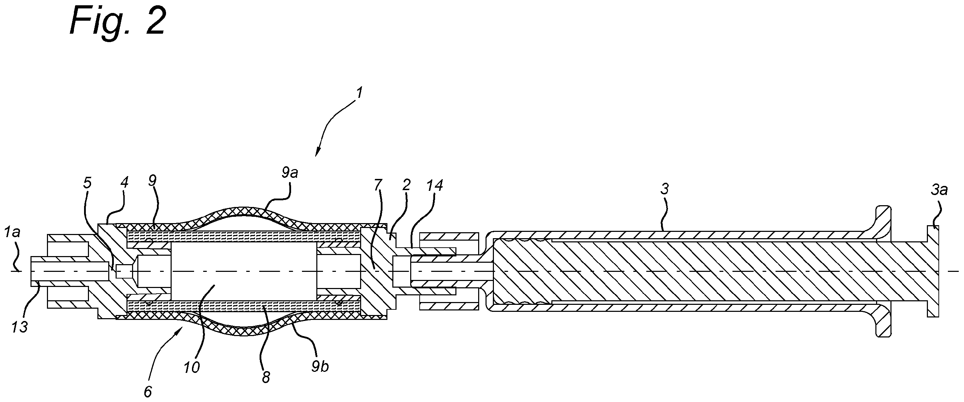

Figure 2 shows an exemplary cross sectional view of an embodiment of a single walled fluid applicator 1 according to the present invention. In the embodiment shown, the inlet end 2 may also comprise an inlet one-way valve 7 to prevent back flow out of the inlet end 2 when, for example, the flexible wall portion 8 is being compressed. Advantageously, the further one-way valve 7 also allows gas (e.g. air) to be drawn into the inner volume 10 when it re-expands once the flexible wall portion 8 is released (and no syringe or other fluid supply device 3 is attached).

In an even further embodiment, a filter (e.g. an anti-bacterial filter such as a HEPA filter) can be attached to the inlet end 2 after removing the fluid supply device 3, in order to maintain a non-contaminated atmosphere inside the fluid applicator 1.

In an advantageous embodiment, the main tubular container body 6 is a flexible or pliable tubular container body 6, so that the fluid applicator 1 can be bent by manual force if needed, e.g. by fingertips, to further increase positioning controllability of a needle element attached to the outlet end 4. For example, when a fluid must be injected into a vitreous chamber during an ophthalmic procedure, the needle element attached to the fluid applicator 1 should be positioned accurately without touching and possibly damaging surrounding tissue. Flexibility or pliability of the main tubular container body 6 may further facilitate steady, precise and accurate manual control of the needle element.

To obtain a fluid tight sealed connection of the main tubular container body 6 to the inlet end 2 and outlet end 4, the flexible tubular container body 6 can be clamped, stretched, glued or press fitted to the inlet end 2 and the outlet end 4. The clamped, stretched, glued or press fitted connection ensures a reliable, leak free attachment when the flexible tubular container body 6 is repeatedly compressed for example.

In an alternative embodiment, the main tubular container body 6 comprises a longitudinally arranged rigid wall portion 9 connecting the inlet end 2 and the outlet end 4, wherein the flexible wall portion 8 is longitudinally arranged between the inlet end 2 and the outlet end 4. Longitudinally in this description is meant as being parallel to a longitudinal axis 1a of the fluid applicator 1 along which the rigid wall portion 9 and the flexible wall portion 8 are arranged. The longitudinally arranged rigid wall portion 9 provides support when holding the fluid applicator 1, providing a familiar pencil-like tactile experience.

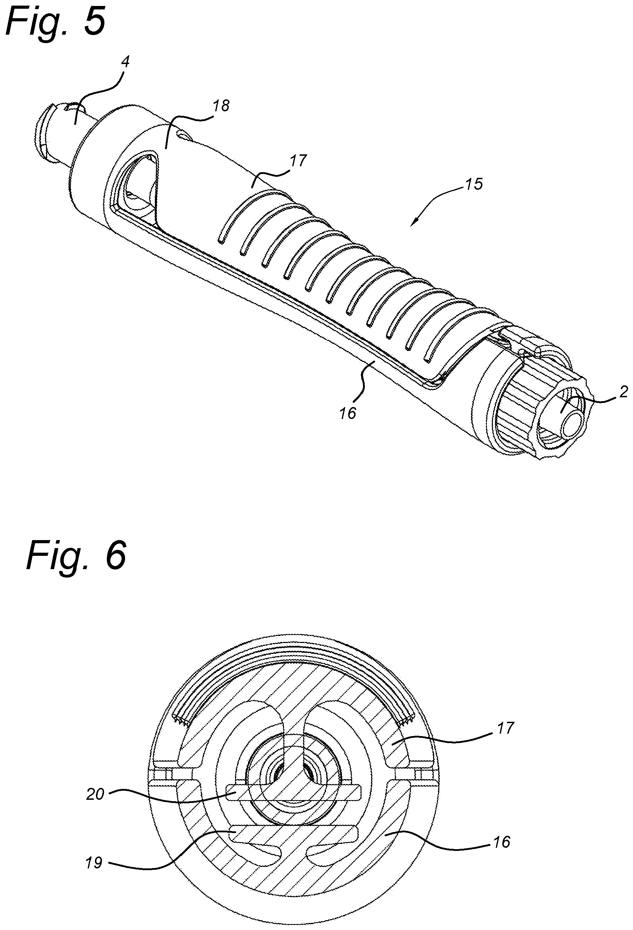

In a further embodiment, the rigid wall portion 9 may compromise two opposing wall sections 9a, 9b, between which the flexible wall portion 8 is arranged. More specifically, the opposing wall sections 9a, 9b may provide further structural rigidity and delimit an area of the fluid applicator 1, in particular the main tubular container body 6, within which the flexible wall portion 8 is accessible and can be manually deformed, compressed, flattened etc. using e.g. fingertips for ejecting a fluid. Alternatively, the rigid wall portion 9 may provide a type of back wall against which the flexible wall portion may be pressed during use from a single side only (see also the embodiment described with reference to Figure 5 below).

According to the present invention, the fluid applicator 1 allows for steady and accurate manual operation comparable to using an ordinary pencil, thereby providing a familiar and natural experience to a user. To that end, controllability of the fluid applicator 1 can be further improved by an embodiment wherein the longitudinally arranged rigid wall portion 9 comprises one or more outward projecting arched portions 9a, 9b. The one or more arched portions 9a, 9b project laterally and provide a local widening of an outer circumference of the main tubular container body 6 for receiving a fingertip. This allows for a more stable handling of the fluid applicator 1 and improves accessibility to the flexible wall portion 8. Furthermore, another advantage of this embodiment is that the one or more outward projecting arched portions 9a, 9b allow the flexible wall portion 8 to expand laterally when it is squeezed and/or flattened by a fingertip.

Note that in an embodiment the two opposing wall sections 9a, 9b mentioned earlier may each comprise an outward projecting arched portion, thereby improving accessibility to the flexible wall portion 8 and to allow lateral expansion of the flexible wall portion 8 when it is compressed by a fingertip.

Since the fluid applicator 1 must allow for mass production, it is desirable that manufacturing costs and engineering complexity are kept to a minimum. In light of this an embodiment is provided wherein the longitudinally arranged rigid wall portion 9, the inlet end 2 as well as the outlet end 4 form a single piece component. The single piece component can be produced economically through injection moulding for example. Since the rigid wall portion 9 may further comprise opposing wall sections 9a, 9b, these wall sections 9a, 9b, the inlet end 2 and outlet end 4 may also form a single piece component.

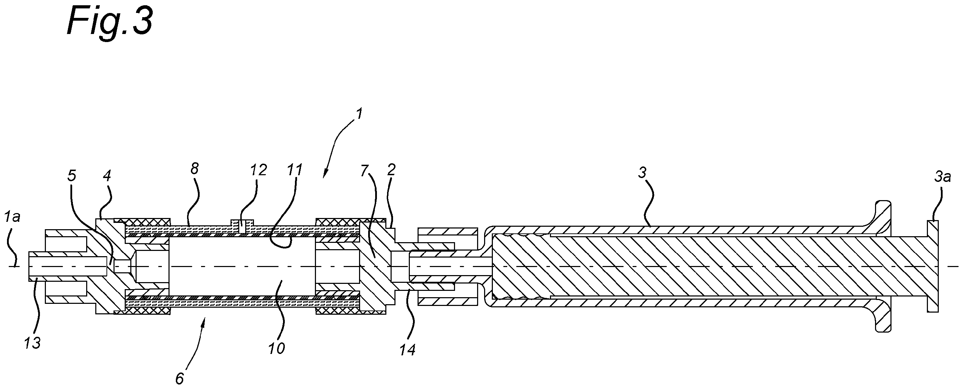

Figure 3 shows a cross section of an embodiment of a double walled fluid applicator according to the present invention. In the embodiment shown, the main tubular container body 6 may further comprise a coaxially arranged flexible or resilient inner tubular container body 11 connecting the inlet end 2 and the outlet end 4, wherein the flexible inner tubular container body 11 comprises the variable inner volume 10. The inner tubular container body 11 provides safe fluid containment and reduces the chance of being punctured as it is contained within and protected by the main tubular container body 6. For further reliability, the inner tubular container body 11 may be clamped, stretched, glued or press fitted to the inlet end 2 and the outlet end 4.

According to the above embodiment, the flexible wall portion 8 of the tubular container body 6 is in engagement with the flexible inner tubular container body 11 when pressed by e.g. a fingertip, thereby reducing the variable inner volume 10 so that a fluid contained therein can be ejected. To facilitate smooth and jitter free ejection of the fluid, frictional engagement between the flexible wall portion 8 and the inner tubular container body 11 may be reduced by providing an annular space between the main tubular container body 6, e.g. the flexible wall portion 8, and the inner tubular container body 11. Such an annular space may reduce the risk of "stick-slip" when the flexible wall portion 8 is in engagement with the inner tubular container body 11, so that smooth fluid ejection from of the fluid applicator 1 is facilitated.

In a further embodiment the annular space may comprises a gas (e.g. air) and can be chosen to minimize, or even prevent, any contact between the flexible wall portion 8 and the inner tubular container body 11. In this embodiment pressing the flexible wall portion 8 pressurizes the gas contained in the annular space, which then compresses the inner tubular container body 11.

In particular situations it may be advantageous to allow outside air into the annular space when removing pressure from the flexible wall portion 8. To achieve this a further embodiment is provided wherein the flexible wall portion 8 of the main tubular container body 6 comprises an aperture 12. The aperture 12 provides a passageway through the flexible wall portion 8 to an annular space between the flexible wall portion 8, i.e. the main tubular container body 6, and the inner tubular container body 11. When the aperture 12 is held open, it allows air from outside the fluid applicator 1 to flow into the annular space, so that a compressed, squeezed or flattened flexible wall portion 8 can return to its neutral shape in rest once the flexible wall portion 8 is being released.

According to the invention the flexible wall portion 8 is arranged for engagement with e.g. a fingertip, wherein the aperture 12 of the flexible wall portion 8 can be closed with the fingertip when a user wishes to eject a fluid from the fluid applicator 1. For example, closing the aperture 12 with a fingertip allows air between the flexible wall portion 8 and inner tubular container body 11 to be compressed. The increasing air pressure between the flexible wall portion 8 and inner tubular container body 11 then compresses the inner tubular container body 11. Conversely, when the aperture 12 in the flexible wall portion 8 is opened by removing the fingertip once fluid ejection has finished, the flexible wall portion 8 expands to a neutral position due to its flexibility and resilience.

During a procedure it may be advantageous to have visual feedback as to the fluid being stored in the fluid applicator 1. An embodiment is therefore provided wherein the inner tubular container body 11 is at least in part transparent, so that visual feedback is given on the amount of fluid and/or what type of fluid is stored in the fluid applicator 1. This may also apply to the flexible wall portion 8, which in an advantageous embodiment is at least in part transparent, thereby providing visual feedback on the fluid being stored in the fluid applicator 1.

As mentioned earlier, the fluid applicator 1 of the present invention provides a pencil like sized applicator providing a natural and familiar handling experience to a user, particularly when handled like a pencil and operated between e.g. fingertips for operating (e.g. squeezing) the flexible wall portion 8. Since there are a variety of procedures in e.g. ophthalmology for which the fluid applicator 1 is needed, the main tubular container body 6 may be adapted to accommodate a different amounts of fluids for a particular procedure.

For example, the fluid application 1 can be adapted for use as a dye applicator or as a perfluorocarbons (PFC) applicator. In case of a dye applicator or for subchoroidal buckling applications (see below), the inner volume 10 in rest may allow for smaller fluid storage of about 0.5 ml, e.g. between 0.3 and 0.7 ml. In case the fluid applicator 1 is used as a PFC applicator, the inner volume 10 in rest may allow for larger fluid storage of about 5 ml, e.g. between 3 ml and 7 ml. So according to the present invention, the variable inner volume 10 may allow for fluid storage between 0.3 ml to 7 ml or even larger if needed, e.g. a maximum volume of 10 ml.

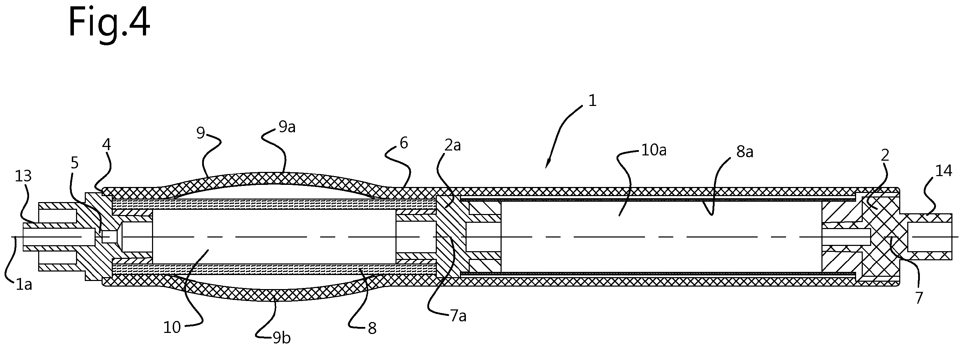

Figure 4 shows a cross sectional view of a further embodiment of the fluid applicator according to the present invention. In the embodiment shown, the main tubular container body 6 may comprise at least in part a further flexible wall portion 8a providing a further variable inner volume 10a to the tubular container body 6. The further flexible wall portion 8a may also be provided as a separate element, e.g. in the form of a flexible tubular bag. An intermediate inlet end 2a is arranged between and fluidly connected to the variable inner volume 10 and the further variable inner volume 10a, thus providing a passageway there between. This allows the further variable inner volume 10a to be supplied first with a fluid through the inlet end 2 when a fluid supply device 3 is connected thereto. Once the further variable inner volume 10a is adequately filled, the fluid supply device 3 can be disconnected. Subsequently, the variable inner volume 10 can be filled with the fluid from the further variable inner volume 10a by pressing on the further flexible wall portion 8a. Alternatively, both the further variable inner volume 10a and variable inner volume 10 may be filled at the same time. In an advantageous embodiment the intermediate inlet end 2a may be closable (e.g. using a one-way valve) to reduce back flow of fluid from the variable inner volume 10 to the further variable inner volume 10a as the flexible wall portion 8 is being compressed for ejecting the fluid from the fluid applicator 1.

During use of the fluid applicator 1 the variable inner volume 10 is arranged to withdraw the fluid from the further variable inner volume 10a through suction for each compression and subsequent release of the flexible wall portion 8. So upon releasing the flexible wall portion 8, an expansion of the variable inner volume 10 allows the fluid in the further variable inner volume 10a to be transferred to the variable inner volume 10 through suction.

As shown in the embodiment of Figure 4, the inlet end 2 comprises the inlet one-way valve 7. In a particular embodiment the inlet end 2 alternatively comprises a connection activated valve 7, meaning that the inlet valve 7 is configured to open upon connection of the inlet end 2 to the fluid supply device 3 and to remain closed upon disconnection thereof. As an alternative a cap is used to close off the inlet end 2 when no fluid supply device 3 is connected. In this way the fluid applicator 1 can be used without the fluid supply device 3 being connected. Furthermore, it allows for a forced opening of the inlet valve 7 only when the fluid supply device 3 is connected to the inlet end 2. When the fluid supply device 3 is disconnected from the fluid applicator 1, the inlet valve 7 remains closed even when, for example, a pressure differential exists over the inlet valve 7. Note that the connection activated inlet valve 7 may be used for all embodiments of the fluid applicator 1, including the embodiments described with reference to Figures 1-3. In an advantageous embodiment, the connection activated inlet valve 7 comprises a Luer type connector which opens the inlet valve 7 upon connection of the Luer type connector to the fluid supply device 3 and to close the inlet valve 7 upon disconnecting the fluid supply device 3.

As mentioned above the intermediate inlet end 2a may be a closable intermediate inlet end 2a. In a further embodiment, the intermediate inlet end 2a comprises an intermediate inlet (one-way) valve 7a. The intermediate inlet one-way valve 7a is e.g. configured to open upon expansion of the variable inner volume 10, so that a fluid from the further variable inner volume 10a can be drawn into the variable inner volume 10. The intermediate inlet one-way valve 7a is further configured to close upon compression of the flexible wall portion 8, i.e. when the variable inner volume 10 is reduced and the fluid is ejected from the fluid applicator 1.

In light of the above, transferring the fluid from the further variable inner volume 10a to the variable inner volume 10 may be further facilitated by providing different grades of flexibility (or pliability) to the variable inner volume 10 and the further variable inner volume 10a. For example, in an embodiment the further flexible wall portion 8a exhibits a higher flexibility (or pliability) than a flexibility (or pliability) of the flexible wall portion 8. This embodiment allows the further flexible wall portion 8a to be deformed much easier that the flexible wall portion 8, so that the further variable inner volume 10a can be reduced upon suction of the fluid therefrom by expansion of the variable inner volume 10.

In a further advantageous embodiment, the flexible wall portion 8 exhibits a higher resiliency than the further flexible wall portion 8a, so that upon releasing the flexible wall portion 8 sufficient suction is created through expansion of the variable inner volume 10, thereby inducing fluid transfer from the further variable inner volume 10a to the variable inner volume 10.

In a practical embodiment, the fluid applicator 1 may further comprise a flexible bag or pouch providing the further variable inner volume 10a, wherein the bag or pouch comprises the further flexible wall portion 8a. The pouch may be envisaged as a thin walled pouch that is easily expanded upon injection of a fluid by the fluid supply device 3 into the pouch, and which is collapsible upon suction of the fluid therefrom. In an embodiment, the flexible bag or pouch is clamped, stretched, glued or press fitted to the inlet end 2 and/or the intermediate inlet end 2a, providing a reliable, leak free connection. Alternatively, a Luer connection may be implemented to connect the pouch.

The embodiment as depicted in Figure 4 also allows for the application of an inner tubular container body 11 as depicted in Figure 3. That is, an embodiment is provided wherein the main tubular container body 6 further comprises a coaxially arranged flexible inner tubular container body comprising the variable inner volume 10 and connecting the intermediate inlet end 2a and the outlet end 4. In an advantageous embodiment the flexible wall portion 8 may also be provided with an aperture 12 allowing closure thereof with a fingertip to compresses the inner tubular container body as the flexible wall portion 8 is squeezed, so that the fluid contained within the inner tubular container body can be ejected. As in the embodiments described above, when the aperture 12 is held open, it allows air from outside the fluid applicator 1 to flow into the annular space, so that a compressed, squeezed or flattened flexible wall portion 8 can return to its neutral shape in rest once the flexible wall portion 8 is being released.

In an embodiment the inner tubular container body may be clamped, stretched, glued or press fitted to the intermediate inlet end 2a and the outlet end 4, providing reliable and leak free connection.

As described above, the present invention embodiments allow for a fluid storage of between 0.3 ml and 10 ml. To allow for such range of storage volumes for the variable inner volume 10 (and the further variable inner volume 10a) for a given circumferential size/diameter of the main tubular container body 6, a longitudinal length of the main tubular container body 6 can be adapted for obtaining a required inner volume 10 and/or further inner volume 10a. Note that changing the longitudinal length of the main tubular container body 6 does not change the pencil-like handling experience of the fluid applicator 1 and does not affect the ability to provide steady and accurate manual control using e.g. one or more finger tips to operate the flexible wall portion 8.

The present invention fluid applicator 1 can also be used in other applications, e.g. for further ophthalmic applications such as the treatment of subchoroidal buckling. In such applications a gel is inserted in specific locations inside the eye between the choroid and the sclera to force back the choroid to the retina surface when a small rupture is present in the retina or when the retina has come off the choroid. A gel can be regarded as a (viscous) fluid, and can be introduced into a fluid applicator 1 according to the present invention. Further elements are specific for this application and may differ from the embodiments discussed above.

Such an embodiment is shown in Figure 5 as a perspective view. The fluid applicator 1 is provided with a squeeze actuator 15. The squeeze actuator 15 is provided around the fluid applicator 1, of which the input end 2 and output end 4 are visible. The squeeze actuator 15 comprises a fluid applicator retaining element 16 and an actuator element 17, the fluid applicator retaining element 16 and actuator element 17 being moveably connected to each other to allow a squeezing action on the flexible wall portion 8. The fluid applicator retaining element 16 and an actuator element 17 are enveloping at least the flexible wall portion 8 of the fluid applicator 1 to allow to exercise a force on the flexible wall portion 8.

The fluid applicator retaining element 16 and actuator element 17 may even be embodied as two similar shaped elements, e.g. resembling tweezer members which can be pressed towards each other.

In an even further embodiment, the squeeze actuator 15 comprises two or even more actuator elements 17, which would allow an easier actuation less dependent on the rotational position of the squeeze actuator 15.

The inlet end 2 can be implemented similar to the embodiments described above, e.g. using a Luer fitting, allowing to fill the inner volume 10 with the gel. In a further embodiment the inlet end 2 may be closed off for actual use of the fluid applicator using a simple cap. The outlet end 4 may be also provided with connecting elements similar to the embodiments described above, e.g. for connection of a needle (of varying size). It is noted that because in this embodiment the fluid is a viscous fluid (gel), the fluid applicator 1 may be usable without having an outlet one-way valve present.

As shown in the perspective view of Figure 5, the fluid applicator retaining element 16 and actuator element 17 are moveably connected to each other by means of a hinge part 18. This would allow to manufacture the squeeze actuator 15 as a single work piece, e.g. using injection moulding or even using 3D printing techniques. A sufficiently strong pressing action while holding the fluid applicator 1 (with needle) in a pen like grip is then easily possible.

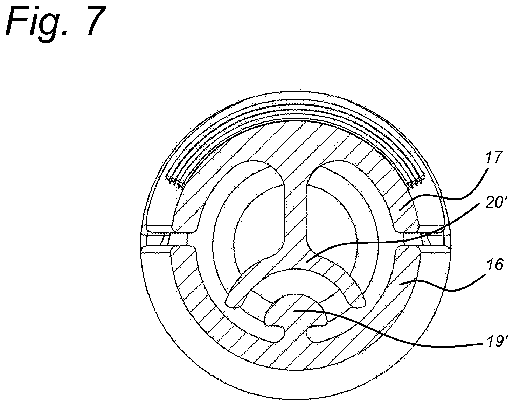

To further enhance the actuating action using the squeeze actuator 15, the fluid applicator retaining element 16 and/or actuator element 17 comprise an inwardly extending pressure element 19, 20; 19', 20'. Two alternative embodiments thereof are shown in the cross sectional views of Figures 6 and 7. In Figure 6, an embodiment is shown wherein both the fluid applicator retaining element 16 and the actuator element 17 are implemented as inwardly positioned T-shaped elongate elements 19, 20, able to efficiently press onto the flexible wall portion 8. In the embodiment shown in the cross sectional view of Figure 7, these elements comprise arcuate shaped elements 19', 20', which are adapted to the cylindrical shape of the flexible wall portion 8 when implemented as a tube. In both embodiments of Figure 6 and 7 the elongate elements 19, 20; 19', 20' are at an angle to each other, allowing the main tubular container body 6 to be squeezed from the side of the inlet end 2 towards the side of the outlet end 4. In this manner, the main tubular container body 6 can be emptied in an efficient manner, even up to complete emptying of the main tubular container body 6, without excessive deformation of the flexible wall portion 8 (e.g. due to the highly viscous gel).

It is noted that the squeeze actuator 15 may also be used in conjunction with the fluid applicator 1 as described with reference to the embodiments relating to the application as a dye applicator or as a perfluorocarbons (PFC) applicator.

The present invention embodiments have been described above with reference to a number of exemplary embodiments as shown in the drawings. Modifications and alternative implementations of some parts or elements are possible, and are included in the scope of protection as defined in the appended claims.

## Claims

1. An ophthalmic fluid applicator comprising:
an inlet end (2) for connection to a fluid supply device (3);
an outlet end (4) for attachment to a needle element, and
a main tubular container body (6) connecting the inlet end (2) and the outlet end (4);
wherein the main tubular container body (6) comprises at least in part a flexible wall portion (8) providing a variable inner volume (10) to the tubular container body (6) and a longitudinally arranged rigid wall portion (9) connecting the inlet end (2) and the outlet end (4), and wherein the flexible wall portion (8) is longitudinally arranged between the inlet end (2) and the outlet end; **characterised in that** the inlet end (2) comprises an inlet one-way valve (5).

2. An ophthalmic fluid applicator according to claim 1, wherein:
the outlet end (4) comprises an outlet one-way valve (5), and/or
the inlet end comprises a connection activated inlet valve (7).

3. An ophthalmic fluid applicator according to any one of claims 1-2, wherein the main tubular container body (6) comprises at least in part a further flexible wall portion (8a) providing a further variable inner volume (10a) to the tubular container body (6), wherein an intermediate inlet end (2a) is arranged between and fluidly connected to the variable inner volume (10) and the further variable inner volume (10a).

4. An ophthalmic fluid applicator according to any one of claims 1-3, wherein the main tubular container body (6) is a flexible tubular container body (6).

5. An ophthalmic fluid applicator according to claim 1, wherein the longitudinally arranged rigid wall portion (9), the inlet end (2) and the outlet end (4) form a single piece component.

6. An ophthalmic fluid applicator according to any of claims 1 to 5, wherein the longitudinally arranged rigid wall portion (9) comprises one or more outward projecting arched portions (9a, 9b).

7. An ophthalmic fluid applicator according to any one of claims 1-6, wherein the main tubular container body (6) further comprises a coaxially arranged flexible inner tubular container body (11) comprising the variable inner volume (10) and connecting the inlet end (2) and the outlet end (4) or connecting the intermediate inlet end 2a and the outlet end (4).

8. An ophthalmic fluid applicator according to claim 7, further comprising an annular space between the main tubular container body (6) and the inner tubular container body (11).

9. An ophthalmic fluid applicator according to any one of claims 7-8, wherein the flexible wall portion (8) of the main tubular container body (6) comprises an aperture (12).

10. An ophthalmic fluid applicator according to any one of claims 7-9, wherein:
the inner tubular container body (11) is at least in part transparent; and/or
the flexible wall portion (8) is at least in part transparent.

11. An ophthalmic fluid applicator according to any one of claims 1-10, wherein the inlet end (2) and/or the outlet end (4) comprises a Luer type connector (13, 14).

12. An ophthalmic fluid applicator according to any one of claims 1-11, wherein the variable inner volume (10) has a maximum volume of 10 ml.

13. An ophthalmic fluid applicator according to any one of claims 1-12 further comprising a squeeze actuator (15), the squeeze actuator (15) comprising a fluid applicator retaining element (16) and an actuator element (17), the fluid applicator retaining element (16) and actuator element (17) being moveably connected to each other to allow a squeezing action on the flexible wall portion (8).

14. An ophthalmic fluid applicator according to claim 13, wherein the fluid applicator retaining element (16) and actuator element (17) are moveably connected to each other by means of a hinge part (18).

15. An ophthalmic fluid applicator according to claim 13 or 14, wherein the fluid applicator retaining element (16) and/or actuator element (17) comprise an inwardly extending pressure element (19, 20; 19', 20').

## Patentansprüche

1. Ophthalmischer Fluidapplikator, aufweisend:
ein Einlassende (2) zur Verbindung mit einer Fluidversorgungsvorrichtung (3);
ein Auslassende (4) zur Befestigung an einem Nadelelement und
einem rohrförmigen Hauptbehälterkörper (6), der das Einlassende (2) und das Auslassende (4) verbindet;
wobei der rohrförmige Hauptbehälterkörper (6) zumindest teilweise einen flexiblen Wandabschnitt (8) umfasst, der ein variables Innenvolumen (10) zum rohrförmigen Behälterkörper (6) bereitstellt, und einen in Längsrichtung angeordneten starren Wandabschnitt (9), der das Einlassende (2) und das Auslassende (4) verbindet, und wobei der flexible Wandabschnitt (8) in Längsrichtung zwischen dem Einlassende (2) und dem Auslassende angeordnet ist; **dadurch gekennzeichnet, dass** das Einlassende (2) ein Einlasseinwegventil (5) umfasst.

2. Ophthalmischer Fluidapplikator nach Anspruch 1, wobei:
das Auslassende (4) umfasst ein Auslasseinwegventil (5), und/oder
das Einlassende umfasst ein verbindungsaktiviertes Einlassventil (7).

3. Ophthalmischer Fluidapplikator nach einem der Ansprüche 1 bis 2, wobei der rohrförmige Hauptbehälterkörper (6) zumindest teilweise einen weiteren flexiblen Wandabschnitt (8a) umfasst, der ein weiteres variables Innenvolumen (10a) für den rohrförmigen Behälterkörper (6) bereitstellt, wobei ein intermediäres Einlassende (2a) zwischen dem variablen Innenvolumen (10) und dem weiteren variablen Innenvolumen (10a) angeordnet und fluidisch verbunden ist.

4. Ophthalmischer Fluidapplikator nach einem der Ansprüche 1 bis 3, wobei der rohrförmige Hauptbehälterkörper (6) ein flexibler rohrförmiger Behälterkörper (6) ist.

5. Ophthalmischer Fluidapplikator nach Anspruch 1, wobei der längs angeordnete starre Wandabschnitt (9), das Einlassende (2) und das Auslassende (4) eine einteilige Komponente bilden.

6. Ophthalmischer Fluidapplikator nach einem der Ansprüche 1 bis 5, wobei der längs angeordnete starre Wandabschnitt (9) einen oder mehrere nach außen vorstehende gewölbte Abschnitte (9a, 9b) umfasst.

7. Ophthalmischer Fluidapplikator nach einem der Ansprüche 1 bis 6, wobei der rohrförmige Hauptbehälterkörper (6) ferner einen koaxial angeordneten flexiblen inneren rohrförmigen Behälterkörper (11) umfasst, der das variable Innenvolumen (10) umfasst und das Einlassende (2) und das Auslassende (4) verbindet oder das Zwischeneinlassende 2a und das Auslassende (4) verbindet.

8. Ophthalmischer Fluidapplikator nach Anspruch 7, ferner umfassend einen ringförmigen Raum zwischen dem rohrförmigen Hauptbehälterkörper (6) und dem rohrförmigen Innenbehälterkörper (11).

9. Ophthalmischer Fluidapplikator nach einem der Ansprüche 7 bis 8, wobei der flexible Wandabschnitt (8) des rohrförmigen Hauptbehälterkörpers (6) eine Öffnung (12) aufweist.

10. Ophthalmischer Fluidapplikator nach einem der Ansprüche 7 bis 9, wobei:
der innere rohrförmige Behälterkörper (11) ist zumindest teilweise transparent; und/oder
der flexible Wandabschnitt (8) ist zumindest teilweise transparent.

11. Ophthalmischer Fluidapplikator nach einem der Ansprüche 1 bis 10, wobei das Einlassende (2) und/oder das Auslassende (4) einen Luer-Anschluss (13, 14) umfasst.

12. Ophthalmischer Fluidapplikator nach einem der Ansprüche 1 bis 11, wobei das variable Innenvolumen (10) ein maximales Volumen von 10 ml aufweist.

13. Ophthalmischer Fluidapplikator nach einem der Ansprüche 1 bis 12, ferner umfassend eine Quetschbetätigungsglied (15), wobei das Quetschbetätigungsglied (15) ein Fluid-Applikator-Halteelement (16) und ein Betätigungselement (17) umfasst, wobei das Fluid-Applikator-Halteelement (16) und das Betätigungselement (17) beweglich miteinander verbunden sind, um eine Quetschwirkung auf den flexiblen Wandabschnitt (8) zu ermöglichen.

14. Ophthalmischer Fluidapplikator nach Anspruch 13, wobei das Fluid-Applikator-Halteelement (16) und das Betätigungselement (17) über ein Scharnierteil (18) beweglich miteinander verbunden sind.

15. Ophthalmischer Fluidapplikator nach Anspruch 13 oder 14, wobei das Fluid-Applikator-Halteelement (16) und/oder das Betätigungselement (17) ein sich nach innen erstreckendes Druckelement (19, 20; 19', 20') umfassen.

## Revendications

1. Applicateur de fluide ophtalmique comprenant :
une extrémité d'entrée (2) destinée à une connexion à un dispositif d'alimentation en fluide (3) ;
une extrémité de sortie (4) destinée à être fixée à un élément d'aiguille, et
un corps de conteneur tubulaire principal (6) connectant l'extrémité d'entrée (2) et l'extrémité de sortie (4) ;
dans lequel le corps de conteneur tubulaire principal (6) comprend au moins en partie une partie de paroi flexible (8) fournissant un volume interne variable (10) au corps de conteneur tubulaire (6) et une partie de paroi rigide agencée longitudinalement (9) connectant l'extrémité d'entrée (2) et l'extrémité de sortie (4), et dans lequel la partie de paroi flexible (8) est agencée longitudinalement entre l'extrémité d'entrée (2) et l'extrémité de sortie, **caractérisé en ce que** l'extrémité d'entrée (2) comprend un clapet anti-retour (5).

2. Applicateur de fluide ophtalmique selon la revendication 1, dans lequel :
l'extrémité de sortie (4) comprend un clapet anti-retour de sortie (5), et/ou
l'extrémité d'entrée comprend une soupape d'admission activée par connexion (7).

3. Applicateur de fluide ophtalmique selon l'une quelconque des revendications 1 et 2, dans lequel le corps de conteneur tubulaire principal (6) comprend au moins en partie une partie de paroi flexible supplémentaire (8a) fournissant un volume intérieur variable supplémentaire (10a) au corps de conteneur tubulaire (6), dans lequel une extrémité d'entrée intermédiaire (2a) est agencée entre le volume intérieur variable (10) et le volume intérieur variable supplémentaire (10a), et connectée à ceux-ci.

4. Applicateur de fluide ophtalmique selon l'une quelconque des revendications 1 à 3, dans lequel le corps de conteneur tubulaire principal (6) est un corps de conteneur tubulaire flexible (6).

5. Applicateur de fluide ophtalmique selon la revendication 1, dans lequel la partie de paroi rigide agencée longitudinalement (9), l'extrémité d'entrée (2) et l'extrémité de sortie (4) forment un composant monobloc.

6. Applicateur de fluide ophtalmique selon l'une quelconque des revendications 1 à 5, dans lequel la partie de paroi rigide agencée longitudinalement (9) comprend une ou plusieurs parties arquées faisant saillie vers l'extérieur (9a, 9b).

7. Applicateur de fluide ophtalmique selon l'une quelconque des revendications 1 à 6, dans lequel le corps de conteneur tubulaire principal (6) comprend en outre un corps de conteneur tubulaire interne flexible agencé coaxialement (11) comprenant le volume intérieur variable (10) et connectant l'extrémité d'entrée (2) et l'extrémité de sortie (4) ou connectant l'extrémité d'entrée intermédiaire (2a) et l'extrémité de sortie (4).

8. Applicateur de fluide ophtalmique selon la revendication 7, comprenant en outre un espace annulaire entre le corps de conteneur tubulaire principal (6) et le corps de conteneur tubulaire intérieur (11).

9. Applicateur de fluide ophtalmique selon l'une quelconque des revendications 7 à 8, dans lequel la partie de paroi flexible (8) du corps de conteneur tubulaire principal (6) comprend une ouverture (12).

10. Applicateur de fluide ophtalmique selon l'une quelconque des revendications 7 à 9, dans lequel :
le corps de conteneur tubulaire intérieur (11) est au moins en partie transparent ; et/ou
la partie de paroi flexible (8) est au moins en partie transparente.

11. Applicateur de fluide ophtalmique selon l'une quelconque des revendications 1 à 10, dans lequel l'extrémité d'entrée (2) et/ou l'extrémité de sortie (4) comprend un connecteur de type Luer (13, 14).

12. Applicateur de fluide ophtalmique selon l'une quelconque des revendications 1 à 11, dans lequel le volume intérieur variable (10) a un volume maximal de 10 ml.

13. Applicateur de fluide ophtalmique selon l'une quelconque des revendications 1 à 12, comprenant en outre un actionneur de compression (15), l'actionneur de compression (15) comprenant un élément de retenue d'applicateur de fluide (16) et un élément d'actionnement (17), l'élément de retenue d'applicateur de fluide (16) et l'élément d'actionnement (17) étant connectés de manière mobile l'un à l'autre pour permettre une action de compression sur la partie de paroi flexible (8).

14. Applicateur de fluide ophtalmique selon la revendication 13, dans lequel l'élément de retenue d'applicateur de fluide (16) et l'élément d'actionnement (17) sont connectés de manière mobile l'un à l'autre au moyen d'une partie d'articulation (18).

15. Applicateur de fluide ophtalmique selon la revendication 13 ou 14, dans lequel l'élément de retenue d'applicateur de fluide (16) et/ou l'élément d'actionnement (17) comprennent un élément de pression s'étendant vers l'intérieur (19, 20 ; 19', 20').
